# EUROPEAN PATENT SPECIFICATION

(11) **EP 3 784 062 B1**
(45) Date of publication and mention of the grant of the patent: **19.01.2022**
(21) Application number: 19718731.3
(22) Date of filing: 24.04.2019
(51) Int. Cl.: A23L 33/15, A23L 33/155, A23L 29/212, A23L 29/219, A23K 20/174, A61K 8/67, A61Q 19/00

(54) **POWDEROUS FORMULATION**
PULVERFÖRMIGE FORMULIERUNG
FORMULATION PULVÉRULENTE

(30) Priority: 27.04.2018 EP 18169734
(43) Date of publication of application: 03.03.2021
(73) Proprietor: DSM IP Assets B.V., 6411 TE Heerlen (NL)
(72) Inventor: BULBARELLO, Andrea, 4303 Kaiseraugst (CH); KIRCHEN, Stefanie, 4303 Kaiseraugst (CH); LINDEMANN, Thomas, 4303 Kaiseraugst (CH)
(74) Representative: Kurt, Manfred
(86) International application number: PCT/EP2019/060491
(87) International publication number: WO 2019/206981

(56) References cited:
- WO-A1-2014/180827
- WO-A1-2017/097974
- WO-A2-2009/010305

## Description

The present invention relates a powderous formulation comprising at least one fat-soluble vitamin, which can be produced easily and which can be used in many fields of application.

Examples of fat-soluble vitamins are vitamin A, D, E and K and derivatives thereof such as vitamin A esters, e.g. vitamin A acetate and palmitate, and vitamin E esters, e.g. tocopherol acetate).

Solid powderous formulation comprising at least one fat-soluble active vitamin need to be stable and easy to be produced. Furthermore, the formulation should also be handled easy, which means the flowability of the formulation should be good.

WO 2014/180827 discloses a powder composition comprising dl-α-tocopherol acetate, pea maltodextrin having a DE of 17 and modified food starch (Capsul HS).

Surprisingly it has been found that a powderous composition comprising at least two different kind of maltodextrins has improved properties.

One surprising advantage of the powderous composition according to the present invention is that the composition is not sticky (even during the drying process, which is usually done by spray-drying). The composition does not stick to the wall of the drying apparatus. Therefore the loss of material during the production is decreased as well as the time for cleaning is shortened. Furthermore the new formulation has also excellent flowability properties.

Therefore, the present invention relates to a powderous composition (I) comprising
(i) up to 70 weight-% (wt-%), based on the total weight of the powderous composition, of at least one fat-soluble vitamin, and
(ii) 5 - 30 wt-%, based on the total weight of the powderous composition, of at least one D-glycose oligomer (preferably a maltodextrin) (GO1) having a DE of < 18, and
(iii) 5 - 30 wt-%, based on the total weight of the powderous composition, of at least one D-glycose oligomer (preferably a maltodextrin) (GO2) having a DE of > 18, and
(iv) 5 - 70 wt-%, based on the total weight of the powderous composition, of at least one modified polysaccharide.

It is clear that the percentages always add up to 100.

The composition according to the present invention is a dry powder. Nevertheless it can also comprise some water, which originates from the emulsion. Usually and preferably, the water content is less than 5 wt-%, based on the total weight of the powderous composition. Usually less than 4wt-%.

Preferably, the at least one fat-soluble vitamin is chosen from the group consisting of vitamin A, D, E and K and their derivatives.

More preferably, the at least one fat-soluble vitamin is chosen from the group consisting of vitamin A esters (such as vitamin A acetate and palmitate), and vitamin E esters, e.g. tocopherol acetate

Therefore, the present invention relates to a powderous composition (II), which is powderous composition (I), wherein the at least one fat-soluble vitamin is chosen from the group consisting of vitamin A, D, E and K and their derivatives.

Therefore, the present invention relates to a powderous composition (II'), which is powderous composition (I) or (II), wherein the at least one fat-soluble vitamin is chosen from the group consisting of vitamin A esters (such as vitamin A acetate and palmitate), and vitamin E esters, e.g. tocopherol acetate (esp. dl-α-tocopherol acetate).

The powderous formulation comprises up to 70 wt-% of the at least one fat soluble vitamin, based on the total weight of the powderous formulation.

The formulation can contain also as less about 0.1 wt-% of the at least one fat soluble vitamin, based on the total weight of the powderous formulation.

Preferably the powderous formulation comprises 1 - 60 wt-% of the at least one fat soluble vitamin, based on the total weight of the powderous formulation.

Therefore, the present invention relates to a powderous composition (III), which is powderous composition (I), (II) or (II'), wherein the formulation comprises 0.1 - 70 wt-% of the at least one fat soluble vitamin, based on the total weight of the powderous formulation.

Therefore, the present invention relates to a powderous composition (III'), which is powderous composition (I), (II) or (II'), wherein the formulation comprises 1 - 60 wt-% of the at least one fat soluble vitamin, based on the total weight of the powderous formulation.

Furthermore, the powderous composition according to the present invention comprises two different kind of D-glycose oligomer (preferably maltodextrin). These two different kind of D-glycose oligomer (preferably maltodextrin) differ in view of their DE value.

Dextrose equivalent (DE) is a measure of the amount of reducing sugars present in a sugar product, relative to glucose, expressed as a percentage on a dry basis. For example, a maltodextrin with a DE of 10 would have 10% of the reducing power of dextrose (which has a DE of 100). Maltose, a disaccharide made of two glucose (dextrose) molecules has a DE of 52, correcting for the water loss in molecular weight when the two molecules are combined (180/342). Sucrose actually has a DE of 0 even though it is a disaccharide, because both reducing groups of the monosaccharides that make it are connected, so there are no remaining reducing groups. For solutions made from starch, it is an estimate of the percentage reducing sugars present in the total starch product.

In all glucose polymers, from the native starch to glucose syrup, the molecular chain begins with a reducing sugar, containing a free aldehyde. As the starch is hydrolyzed, the molecules become shorter and more reducing sugars are present. Because different reducing sugars (e.g. fructose and glucose) have different sweetness, it is incorrect to assume that there is any direct relationship between DE and sweetness.

The DE describes the degree of conversion of starch to dextrose:
starch is close to DE = 0,
glucose/dextrose is DE = 100 (percent).

The standard method of determining DE is the Lane-Eynon titration, based on the reduction of copper(II) sulfate in an alkaline tartrate solution, an application of Fehling's test.

For the present invention the first D-glycose oligomer (preferably maltodextrin) (or mixture of them), which is the ingredient (ii) of the formulation and defined as (GO1), has a DE of less than 18, preferably less than 15, more preferably less than 12, even more preferably less than 10.

Most preferably the first D-glycose oligomer (preferably maltodextrin) (or mixture of them), which is the ingredient (ii) of the formulation, has a DE of 2 - 10.

Therefore, the present invention relates to a powderous composition (IV), which is powderous composition (I), (II), (II'), (III) or (III'), wherein (GO1) or mixture of (GO1)s has a DE of less than 15.

Therefore, the present invention relates to a powderous composition (IV'), which is powderous composition (I), (II), (II'), (III) or (III'), wherein (GO1) or mixture of (GO1)s has a DE of less than 12.

Therefore, the present invention relates to a powderous composition (IV"), which is powderous composition (I), (II), (II'), (III) or (III'), wherein (GO1) or mixture of (GO1)s has a DE of less than 10.

Therefore, the present invention relates to a powderous composition (VI‴), which is powderous composition (I), (II), (II'), (III) or (III'), wherein (GO1) or mixture of (GO1)s has a DE of 2 - 10.

For the present invention the second D-glycose oligomer (preferably maltodextrin) (or mixture of them), which is the ingredient (iii) of the formulation and defined as (GO2), has a DE of more than 18, preferably more than 20.

Most preferably the second D-glycose oligomer (or mixture of them), which is the ingredient (iii) of the formulation, has a DE of 20 - 45.

Therefore, the present invention relates to a powderous composition (V), which is powderous composition (I), (II), (II'), (III), (III'), (IV), (IV'), (IV'), (IV") or (IV‴), wherein (GO2) or mixture of (GO2)s has a DE of more than 20.

Therefore, the present invention relates to a powderous composition (V'), which is powderous composition (I), (II), (II'), (III), (III'), (IV), (IV'), (IV'), (IV") or (IV‴), wherein (GO2) or mixture of (GO2)s has a DE of 20 - 45.

The (GO1) and (GO2) can be used in the same amounts (1:1 mixture) as well as in different amounts according to their definition of amounts as described above.

The composition according to present invention comprises at least one modified polysaccharide.

Preferably the modified polysaccharide is modified starch.

Therefore the present invention relates to a powderous composition (VI), which is powderous composition (I), (II), (II'), (III), (III'), (IV), (IV'), (IV'), (IV"), (IV‴), (V) or (V'), wherein the modified polysaccharide is modified starch.

Preferably the modified polysaccharide is of formula (I) 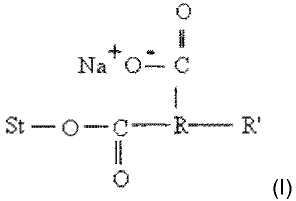 wherein
St is a starch,
R is an alkylene group and R' is a hydrophobic group.

Therefore the present invention relates to a powderous composition (VI'), which is powderous composition (I), (II), (II'), (III), (III'), (IV), (IV'), (IV'), (IV"), (IV‴), (V) or (V'), wherein the modified polysaccharide is of formula (I) 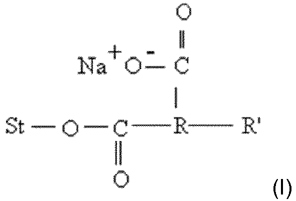 wherein
St is a starch,
R is an alkylene group and R' is a hydrophobic group.

Preferably the modified polysaccharide is starch sodium octenyl succinate.

Therefore the present invention relates to a composition (VI"), which is powderous composition (I), (II), (II'), (III), (III'), (IV), (IV'), (IV'), (IV"), (IV‴), (V) or (V'), wherein the modified polysaccharide is starch sodium octenyl succinate.

Preferably, the powderous composition (I), (II), (II'), (III), (III'), (IV), (IV'), (IV'), (IV"), (IV‴), (V), (V'), (VI), (VI') and (VI") according to present invention has an average inner particle size D [0,5] (inner phase) of less than 500 nm, preferably 150nm - 450nm.

All the sizes of the inner phase D [0,5] in the context of the present patent application were determined by using a Mastersizer 2000. The particle size of the inner phase was determined after redispersing the powderous composition in water.

Therefore the present invention relates to a powderous composition (VII), which is powderous composition (I), (II), (II'), (III), (III'), (IV), (IV'), (IV'), (IV"), (IV‴), (V), (V'), (VI), (VI') or (VI"), wherein the powderous composition has an average inner particle size D [0,5] (inner phase) of less than 500 nm.

Therefore the present invention relates to a powderous composition (VII'), which is powderous composition (I), (II), (II'), (III), (III'), (IV), (IV'), (IV'), (IV"), (IV‴), (V), (V'), (VI), (VI') or (VI"), wherein the powderous composition has an average inner particle size D [0,5] (inner phase) of 150nm - 450nm.

Preferably the powderous composition according to the present invention is a spray dried composition. Other method of production can also be used.

Therefore, the present invention relates to a composition (VIII), which is powderous composition (I), (II), (II'), (III), (III'), (IV), (IV'), (IV'), (IV"), (IV‴), (V), (V'), (VI), (VI'), (VI"), (VII) or (VII'), wherein the powderous composition is a spray dried composition.

The powderous composition according to the present invention can be produced by using technologies known to a person skilled in the art.

In first step an emulsion comprising all ingredients ((i) - (iv)) and water is produced, which is then dried (usually and preferably by spray drying). The water content of the powderous composition depends on the conditions of the applies drying process.

One advantage of the powderous composition according to the present invention is that during the spray-drying procedure, the powderous composition is not sticky and therefore the powderous composition does not stick to the wall of the spray drying tower and therefor the loss of the powderous composition during the drying process is very low and the effort to clean the drying apparatus is lowered.

Therefore the present invention relates to process for production of powderous composition (I), (II), (II'), (III), (III'), (IV), (IV'), (IV'), (IV"), (IV‴), (V), (V'), (VI), (VI'), (VI"), (VII), (VII') or (VIII), characterized in that in a first step an emulsion comprising all ingredients (i) - (iv) and water is produced, which is then in a second step dried to form a powderous composition.

The powderous compositions (I), (II), (II'), (III), (III'), (IV), (IV'), (IV'), (IV"), (IV‴), (V), (V'), (VI), (VI'), (VI"), (VII), (VII') and (VIII) according to present invention are used in food, feed and/or personal care formulations.

Preferably the powderous compositions (I), (II), (II'), (III), (III'), (IV), (IV'), (IV'), (IV"), (IV‴), (V), (V'), (VI), (VI'), (VI"), (VII), (VII') and (VIII) are used in a liquid formulation, preferably in a beverage.

Furthermore, the present invention relates to food, feed and personal care formulations comprising at least one powderous composition (I), (II), (II'), (III), (III'), (IV), (IV'), (IV'), (IV"), (IV‴), (V), (V'), (VI), (VI'), (VI"), (VII), (VII') and/or (VIII).

These formulations can be in any form. Solid, liquid or gel-like.

Preferred are liquid food, feed and personal care formulation comprising at least one powderous composition (I), (II), (II'), (III), (III'), (IV), (IV'), (IV'), (IV"), (IV‴), (V), (V'), (VI), (VI'), (VI"), (VII), (VII') and/or (VIII).

More preferred are beverages comprising at least one powderous composition (I), (II), (II'), (III), (III'), (IV), (IV'), (IV'), (IV"), (IV‴), (V), (V'), (VI), (VI'), (VI"), (VII), (VII') and/or (VIII).

Therefore, a further embodiment of the present invention relates to food, feed and personal care formulations comprising at least one powderous composition (I), (II), (II'), (III), (III'), (IV), (IV'), (IV'), (IV"), (IV‴), (V), (V'), (VI), (VI'), (VI"), (VII), (VII') and/or (VIII).

Therefore, a further embodiment of the present invention relates to liquid food, feed and personal care formulations comprising at least one powderous composition (I), (II), (II'), (III), (III'), (IV), (IV'), (IV'), (IV"), (IV‴), (V), (V'), (VI), (VI'), (VI"), (VII), (VII') and/or (VIII).

Therefore, a further embodiment of the present invention relates to beverages comprising at least one powderous composition (I), (II), (II'), (III), (III'), (IV), (IV'), (IV'), (IV"), (IV‴), (V), (V'), (VI), (VI'), (VI"), (VII), (VII') and/or (VIII).

The concentration of the powderous composition in the food, feed and/or personal care formulations depends on the kind of these formulations.

### Figures:

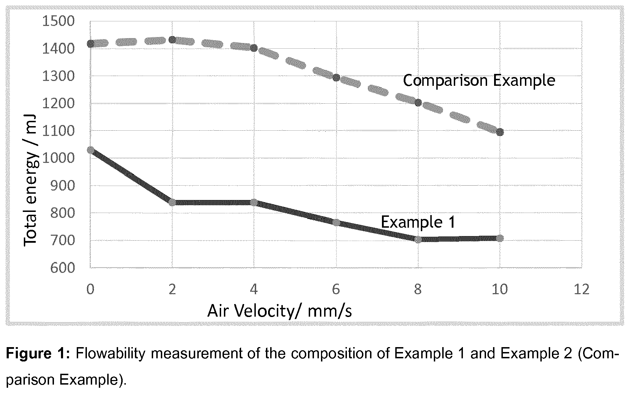

**Figure 1**: Flowability measurement of the composition of Example 1 and Example 2 (Comparison Example).

The invention is illustrated by the following Examples. All temperatures are given in °C and all parts and percentages are related to the weight.

### Example 1 (dl-α-tocopherolacetate):

109 g Capsul HS (Modified Food Starch), 54.6 g of Glucidex 6 (Maltodextrin with a DE ranging between 5 and 8) and 54.6 g of Glucodry 210 (Maltodextrin with a DE ranging between 20 and 23) were put into a 1.5 I reaction vessel and were dispersed in 185 g of deionised water at room temperature. The temperature was increased to 65°C under stirring with a micer disk (2000 rpm, d=6 cm). The solution was kept at 65°C for 60 min.

239.6 g dl-alpha-tocopherolacetate was preheated to 65°C and added within 5-10 min to the mixture of Capsul HS, Glucidex 6 and Glucodry 210 and water under homogenizing with the micer disk (6000 rpm). Simultaneously 91 g water was added to the emulsion within 5-10 min. This emulsion was homogenized for another 30 min. at 65°C with the micer disk (6000 rpm).

Afterwards this emulsion was dried in a spray drying process (emulsion temperature: 65°C). The temperature at the inlet of the spray drying tower was between 180 - 183°C and the temperature at the outlet of the spray drying tower was between 90 - 100°C.

A powderous composition was obtained with a residual moisture content ≤ 3.0%. The size of the inner phase D[0,5] was 380 nm.

The following table shows the amounts of the ingredients of the composition (the values are recalculated for a water-free composition).

**Table 1: composition of the formulation of Example 1**

| **Ingredient** | **Amount [%]** |
|---|---|
| Capsul HS | 23.8 |
| Glucidex 6 | 11.9 |
| Glucodry 210 | 11.9 |
| dl-α-tocopherolacetate | 52.4 |

### Example 2: (Comparison Example)

109 g Capsule HS (Modified Food Starch), 109 g of Glucodry 210 (Maltodextrin with a DE ranging between 20 and 23) were put into a 1.5 I reaction vessel and were dispersed in 185 g of deionised water at room temperature. The temperature was increased to 65°C under stirring with a micer disk (2000 rpm, d=6 cm). The solution was kept at 65°C for 60 min. 239.6 g dl-alpha-tocopherolacetate was preheated to 65°C and added within 5-10 min to the mixture of Cleargum, Glucodry 210, and water under homogenizing with the micer disk (6000 rpm). Simultaneously 91 g water was added to the emulsion within 5-10 min. This emulsion was homogenized for another 30 min. at 65°C with the micer disk (6000 rpm).

Afterwards this emulsion was dried in a spray drying process (emulsion temperature: 65°C). The temperature at the inlet of the spray drying tower was between 180 - 183 °C and the temperature at the outlet of the spray drying tower was between 90 - 100°C.

A powderous composition was obtained with a residual moisture content ≤ 3.0%. The size of the inner phase D[0,5] was 380 nm.

The following table shows the amounts of the ingredients of the composition (the values are recalculated for a water-free composition).

**Table 2: composition of the formulation of Example 2**

| **Ingredient** | **Amount [%]** |
|---|---|
| Capsul HS | 23.8 |
| Glucodry 210 | 23.8 |
| dl-α-tocopherolacetate | 52.4 |

### Example 3: (Vitamin A Acetate)

155 g Cleargum C OA1 (Modified Food Starch), 76.1 g of Glucidex 6, 76.1 g of Glucodry 210 (Maltodextrin with a DE ranging between 20 and 23), and 7 g of Sodium Ascorbate were put into a 1.5 I reaction vessel and were dispersed in 265 g of deionised water at room temperature. The temperature was increased to 65°C under stirring with a micer disk (2000 rpm, d=6 cm). The solution was kept at 65°C for 60 min. 35 g of Vitamin A Acetate and 3.5 g of dl-α-Tocopherol were preheated to 65°C and added within 5-10 min to the mixture of Capsul HS, Glucodry 210, Sodium Ascorbate and water under homogenizing with the micer disk (6000 rpm). This emulsion was homogenized for another 30 min. at 65°C with the micer disk (6000 rpm).

Afterwards this emulsion was dried in a spray drying process (emulsion temperature: 65°C). The temperature at the inlet of the spray drying tower was between 180 - 183 °C and the temperature at the outlet of the spray drying tower was between 90 - 100°C.

A powderous composition was obtained with a residual moisture content ≤ 3.0%. The size of the inner phase D[0,5] was 300 nm.

The following table shows the amounts of the ingredients of the composition (the values are recalculated for a water-free composition).

**Table 3: composition of the formulation of Example 3**

| **Ingredient** | **Amount [%]** |
|---|---|
| Cleargum | 43.5 |
| Glucidex 6 | 21.7 |
| Glucodry 210 | 21.7 |
| Sodium Ascorbate | 2 |
| Vitamin A Acetate | 10.1 |
| dl-α-tocopherolacetate | 1 |

To show how the flowability of the composition according to the present invention was improved, the flowability was measured as described in the following.

### Flowability measurement

The FT4 Powder Rheometer^{®} (Freeman Instruments, UK) is an instrument able to characterize the rheology, or flow properties, of powders. One of the test that the above-mentioned instrument can perform is the so called "Aeration Test", where the cohesive forces of the analyzed powder are quantified. Cohesive forces are a combination of Van der Waal's and electrostatics, and tend to "bond" particles together. Therefore, the higher the measured cohesive forces are, the less flowable the analyzed powder is.

The measured resistance to flow, the Aerated Energy (AE), quantifies the strength of the cohesive forces.

For powders with weak cohesive forces, the Aerated Energy tends towards zero as the powder becomes fully aerated. Powders with moderate to high cohesion will exhibit a reduction in flow energy when aerated, but to a much lesser extent. In these cohesive powders, the tensile forces are too strong for the air to overcome and the particles do not separate.

The composition of Example 1 did show a total Aerated Energy value of 750 mJ when the air velocity was set at 6 mm/s while the composition of the comparison Example (Example 2) did show a value of 1300 at the same air velocity conditions.

The results are to be seen also in the graph.

### Method:

A standard powder volume is poured into a specially designed sample holder (Freeman Technology, UK). The sample holder features a porous bottom, which allows air to pass through and aerate the powder contained in the cylindrical sample holder, in a controlled way.

After sample preparation, the air flow is initiated and a rotating propeller is lowered into the aerated powder.

A specific hardware unit is then providing increasing air speed.

## Claims

1. A powderous composition (I) comprising
(i) up to 70 weight-% (wt-%), based on the total weight of the powderous composition, of at least one fat-soluble vitamin, and
(ii) 5-30 wt-%, based on the total weight of the powderous composition, of at least one D-glycose oligomer (preferably a maltodextrin) (GO1) having a DE of < 18, and
(iii) 5-30 wt-%, based on the total weight of the powderous composition, of at least one D-glycose oligomer (preferably a maltodextrin) (GO2) having a DE of > 18, and
(iv) 5-70 wt-%, based on the total weight of the powderous composition, of at least one modified polysaccharide.

2. Powderous according to claim 1, wherein the at least one fat-soluble vitamin is chosen from the group consisting of vitamin A, D, E and K and their derivatives.

3. Powderous according to any of the preceding claims, wherein the powderous formulation comprises 0.1 - 70 wt-% of the at least one fat soluble vitamin, based on the total weight of the powderous formulation.

4. Powderous according to any of the preceding claims, wherein (GO1) or mixture of (GO1)s has a DE of less than 15.

5. Powderous according to any of the preceding claims, wherein (GO1) or mixture of (GO1)s has a DE of 2 - 10.

6. Powderous according to any of the preceding claims, wherein (GO2) or mixture of (GO2)s has a DE of more than 20.

7. Powderous according to any of the preceding claims, wherein (GO2) or mixture of (GO2)s has a DE of 20 - 45.

8. Powderous according to any of the preceding claims, wherein the modified polysaccharide is modified starch.

9. Powderous according to any of the preceding claims, wherein the modified polysaccharide is of formula (I) 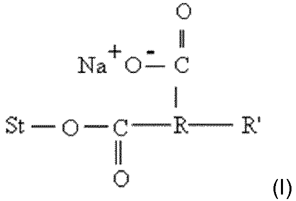 wherein
St is a starch,
R is an alkylene group and R' is a hydrophobic group.

10. Food, feed and personal care formulations comprising at least one powderous composition according to any of the preceding claims.

## Patentansprüche

1. Pulverförmige Zusammensetzung (I), umfassend:
(i) bis zu 70 Gewichtsprozent (Gew.-%), bezogen auf das Gesamtgewicht der pulverförmigen Zusammensetzung, mindestens eines fettlöslichen Vitamins und
(ii) 5 - 30 Gew.-%, bezogen auf das Gesamtgewicht der pulverförmigen Zusammensetzung, mindestens eines D-Glukose-Oligomers (bevorzugt ein Maltodextrin) (GO1), das einen DE-Wert von < 18 aufweist, und
(iii) 5 - 30 Gew.-%, bezogen auf das Gesamtgewicht der pulverförmigen Zusammensetzung, mindestens eines D-Glukose-Oligomers (bevorzugt ein Maltodextrin) (GO2), das einen DE-Wert von > 18 aufweist, und
(iv) 5 - 70 Gew.-%, bezogen auf das Gesamtgewicht der pulverförmigen Zusammensetzung, mindestens eines modifizierten Polysaccharids.

2. Pulverförmige nach Anspruch 1, wobei das mindestens eine fettlösliche Vitamin aus der Gruppe der Vitamine A, D, E und K und ihrer Derivate ausgewählt ist.

3. Pulverförmige nach einem der vorhergehenden Ansprüche, wobei die pulverförmige Formulierung 0,1-70 Gew.-% des mindestens einen fettlöslichen Vitamins, bezogen auf das Gesamtgewicht der pulverförmigen Formulierung, umfasst.

4. Pulverförmige nach einem der vorhergehenden Ansprüche, wobei (GO1) oder eine Mischung von (GO1) einen DE-Wert von weniger als 15 aufweist.

5. Pulverförmige nach einem der vorhergehenden Ansprüche, wobei (GO1) oder eine Mischung von (GO1) einen DE-Wert von 2 - 10 aufweist.

6. Pulverförmige nach einem der vorhergehenden Ansprüche, wobei (GO2) oder eine Mischung von (GO2) einen DE-Wert von mehr als 20 aufweist.

7. Pulverförmige nach einem der vorhergehenden Ansprüche, wobei (GO2) oder eine Mischung von (GO2) einen DE-Wert von 20 - 45 aufweist.

8. Pulverförmige nach einem der vorhergehenden Ansprüche, wobei das modifizierte Polysaccharid modifizierte Stärke ist.

9. Pulverförmige nach einem der vorhergehenden Ansprüche, wobei das modifizierte Polysaccharid die Formel (I) aufweist, 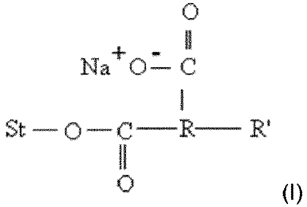 wobei
St eine Stärke ist,
R eine Alkylengruppe und R' eine hydrophobe Gruppe ist.

10. Lebensmittel-, Futtermittel- und Körperpflegeformulierungen, die mindestens eine pulverförmige Zusammensetzung nach einem der vorhergehenden Ansprüche umfassen.

## Revendications

1. Composition en poudre (I) comprenant
(i) jusqu'à 70 % en poids (% en poids), sur la base du poids total de la composition en poudre, d'au moins une vitamine liposoluble, et
(ii) 5 à 30 % en poids, sur la base du poids total de la composition en poudre, d'au moins un oligomère de D-glycose (préférablement une maltodextrine) (GO1) possédant un DE de < 18, et
(iii) 5 à 30 % en poids, sur la base du poids total de la composition en poudre, d'au moins un oligomère de D-glycose (préférablement une maltodextrine) (GO2) possédant un DE de > 18, et
(iv) 5 à 70 % en poids, sur la base du poids total de la composition en poudre, d'au moins un polysaccharide modifié.

2. En poudre selon la revendication 1, l'au moins une vitamine liposoluble étant choisie dans le groupe constitué par la vitamine A, la vitamine D, la vitamine E et la vitamine K et leurs dérivés.

3. En poudre selon l'une quelconque des revendications précédentes, la formulation en poudre comprenant 0,1 à 70 % en poids de l'au moins une vitamine liposoluble, sur la base du poids total de la formulation en poudre.

4. En poudre selon l'une quelconque des revendications précédentes, (GO1) ou un mélange de plusieurs (GO1) possédant un DE inférieur à 15.

5. En poudre selon l'une quelconque des revendications précédentes, (GO1) ou un mélange de plusieurs (GO1) possédant un DE de 2 à 10.

6. En poudre selon l'une quelconque des revendications précédentes, (GO2) ou un mélange de plusieurs (GO2) possédant un DE supérieur à 20.

7. En poudre selon l'une quelconque des revendications précédentes, (GO2) ou un mélange de plusieurs (GO2) possédant un DE de 20 à 45.

8. En poudre selon l'une quelconque des revendications précédentes, le polysaccharide modifié étant un amidon modifié.

9. En poudre selon l'une quelconque des revendications précédentes, le polysaccharide modifié étant de formule (I) 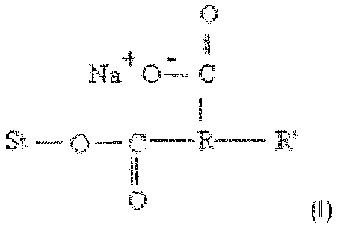
St étant un amidon,
R étant un groupe alkylène et R' étant un groupe hydrophobe.

10. Formulations d'aliment, d'aliment pour animaux et de soin personnel comprenant au moins une composition en poudre selon l'une quelconque des revendications précédentes.
